Europäisches Patentamt

European Patent Office

Office européen des brevets

(19)

(11) Veröffentlichungsnummer : **0 356 647 B1**

(12) **EUROPÄISCHE PATENTSCHRIFT**

(45) Veröffentlichungstag der Patentschrift :
**22.04.92 Patentblatt 92/17**

(51) Int. Cl.$^5$ : **A61F 2/24**

(21) Anmeldenummer : **89111661.8**

(22) Anmeldetag : **27.06.89**

(54) **Herzklappenprothese.**

(30) Priorität : **25.08.88 DE 3828830**

(43) Veröffentlichungstag der Anmeldung :
**07.03.90 Patentblatt 90/10**

(45) Bekanntmachung des Hinweises auf die
Patenterteilung :
**22.04.92 Patentblatt 92/17**

(84) Benannte Vertragsstaaten :
**AT BE CH DE ES FR GB GR IT LI LU NL SE**

(56) Entgegenhaltungen :
**EP-A- 0 275 951**
**WO-A-86/05383**
**GB-A- 2 084 299**

(73) Patentinhaber : **B. Braun Melsungen AG**
**Carl-Braun Strasse**
**W-3508 Melsungen (DE)**

(72) Erfinder : **Knoch, Martin, Dr.-Ing.**
**Kullenhofwinkel 34**
**W-5100 Aachen (DE)**
Erfinder : **Reul, Helmut, Prof. Dr.-Ing.**
**Akazienstrasse 65**
**W-5160 Düren (DE)**
Erfinder : **Rau, Günter, Prof. Dr. rer.nat.**
**Fuchserde 50**
**W-5100 Aachen (DE)**

(74) Vertreter : **Selting, Günther, Dipl.-Ing. et al**
**Patentanwälte von Kreisler, Selting, Werner**
**Deichmannhaus am Hauptbahnhof**
**W-5000 Köln 1 (DE)**

## Beschreibung

Die Erfindung betrifft eine Herzklappenprothese der im Oberbegriff des Patentanspruchs 1 angegebenen Art.

Eine derartige Herzklappenprothese ist aus DE 37 01 755 C1 bekannt. Die bekannte Herzklappenprothese weist einen Klappenring auf, dessen Innenfläche sich über ihre gesamte Länge bis zum Ende des Klappenringes in Durchströmrichtung stetig zunehmend verengt. In dem Klappenring ist mindestens ein Schließkörper schwenkbar gelagert. Dieser Schließkörper besteht aus einer schwenkbaren Klappe, die entlang der Drehachse Lagerzapfen aufweist, welche in Ausnehmungen der Innenwand des Klappenringes gelagert sind.

Bei Herzklappenprothesen besteht die Gefahr, daß sich insbesondere im Bereich der Lagerzapfen der Schließkörper Totwassergebiete bilden, in denen die Gefahr der Thrombenbildung besteht. Aus diesem Grund ist dafür zu sorgen, daß sich in den Ausnehmungen und an den Lagerzapfen keine Blutkörperchen festsetzen kann. Eine weitere Schwierigkeit bei einer Herzklappenprothese, bei der der Querschnitt des Klappenringes sich in Strömungsrichtung verändert, besteht darin, daß die Schließkörper einerseits in der Schließstellung die Öffnung des Klappenringes dicht verschließen, andererseits aber auch klemmfrei in die Öffnungsstellung geschwenkt werden können. Es sind Herzklappenprothesen bekannt, bei denen der Drehbewegung der Schließkörper gleichzeitig eine translatorische Bewegung überlagert ist. Eine solche Kombination von Drehbewegung und translatorischer Bewegung ist bei Klappenringen, deren Querschnitt sich in Strömungsrichtung verändert, aber nicht möglich, weil dadurch entweder die Klemmfreiheit oder die Dichtigkeit beeinträchtigt würde. Wenn dagegen die Lagerzapfen und die entsprechenden Ausnehmungen zylindrisch ausgebildet sind, setzen sich in dem zwischen ihnen vorhandenen Ringraum Blutkörperchen fest, mit der Gefahr der Thrombenbildung.

Aus der Druckschrift GB-A-2 084 299 ist Hersklappenprothese mit einem Klappenring und einem Schließkörper bekannt, wobei der Schließkörper Lagerzapfen aufweist, welche in Ausnehmungen in Form eines Kreissektors mit abgerundetem Innenbereich eingreifen. Die Lagerzapfen haben die Form eines Segmentes einer dünnen Zylinderscheibe mit abgerundeten Übergängen zwischen der flachen oberen Stirnfläche, der schmalen zylindrischen Umfangläche und der flachen unteren Stirnfläche.

Der Erfindung liegt die Aufgabe zugrunde, eine Herzklappenprothese der im Oberbegriff des Patentanspruchs 1 angegebenen Art zu schaffen, die bei guter Abdichtung in der Schließstellung eine klemmfreie Bewegung des Schließkörpers ermöglicht und bei der die Lagerzapfen in den Ausnehmungen Bewegungen ausführen, durch die bei jeder Schließkörperbewegung ein Auswischen des sich in den Ausnehmungen befindenden Blutes erfolgt.

Zur Lösung dieser Aufgabe dienen erfindungsgemäß die im kennzeichnenden Teil des Patentanspruchs 1 angegebenen Merkmale.

Bei der erfindungsgemäßen Herzklappenprothese sind die Lagerzapfen länglich. Sie weisen an den entgegengesetzten Enden Stützflächen auf, wobei die erste Stützfläche in der Schließstellung wirksam ist, um den Schließkörper fest gegen die Innenfläche des Klappenringes zu drücken, und von denen die zweite Stützfläche die Abstützung des Schließkörpers in der Öffnungsstellung gegen den Druck des strömenden Blutes übernimmt. Der längliche Lagerzapfen ist in einer sektorförmigen Ausnehmung untergebracht, in der er sich während der Öffnungs- und Schließbewegungen nach Art eines Scheibenwischers bewegt, um das in dieser Ausnehmung enthaltene Blut auszuwischen. Damit gelingt es, für jeden Schließkörper eine definierte Drehachse einzuhalten, deren Lage sich während der Öffnungs- und Schließbewegungen nicht verändert, und gleichzeitig eine Abstützung des Lagerzapfens in jeder Schwenkstellung an beiden Enden sicherzustellen. Innerhalb der Ausnehmung führt der längliche Lagerzapfen Schwenkbewegungen aus. In der Schließstellung wird der Rand des Schließkörpers an die entgegen der Durchflußrichtung divergierende Innenfläche des Klappenringes gedrückt, ohne daß beim Öffnungsvorgang Klemmungen eintreten würden.

Wenn ein Schließkörper in einem Klappenring gelagert ist, dessen Querschnittsfläche sich in Durchflußrichtung verringert, dann entstehen zwischen dem Schließkörper und der Innenfläche des Ringes einander überlappende Schnittflächen, die es erforderlich machen, Aussparungen an dem Ring oder am Schließkörper vorzusehen. Diese Aussparungen sollten zur Erzielung laminarer Strömungsverhältnisse möglichst gering gehalten werden. Dies gelingt nach Anspruch 2 dadurch, daß die Tangenten an die Innenfläche des Klappenringes und an die Umfangskante des Schließkörpers in der Drehachse zusammenfallen.

Der Radius der zweiten Stützfläche, die am Umfangsbereich des Kreissektors angreift, ist wesentlich größer als der Radius der ersten Stützfläche, die am abgerundeten Innenbereich des Kreissektors angreift. Andererseits kann die Größe der zweiten Stützfläche kleiner gemacht werden als diejenige der ersten Stützfläche, weil die zweite Stützfläche, die während der Öffnungsstellung des Schließkörpers wirksam ist, geringeren Stützkräften ausgesetzt ist. Durch geeignete Bemessung der Größen der beiden Stützflächen können die örtlichen Flächenpressungen dieser Stützflächen gering gehalten werden, mit der Folge, daß die Herzklappenprothese eine lange Lebensdauer hat.

Die kreissektorförmige Ausnehmung zur Aufnahme des Lagerzapfens hat weiterhin den Vorteil, daß diese Ausnehmung einen der Überschneidungsbereiche aufnehmen kann, in denen, wenn die Ausnehmung nicht vorhanden wäre, der Rand des Schließkörpers mit der Innenseite des Klappenringes kollidieren würde.

Im folgenden wird unter Bezugnahme auf die Zeichnungen ein Ausführungsbeispiel der Erfindung näher erläutert.

Es zeigen:

Fig. 1 einen Längsschnitt durch die Herzklappenprothese,

Fig. 2 eine schematische Darstellung einer Lagerstelle aus Richtung des Pfeiles II von Fig. 1,

Fig. 3 eine Stirnansicht eines Schließkörpers mit Lagerzapfen,

Fig. 4 einen Schnitt durch den Klappenring entlang der Linie III-III von Fig. 1,

Fig. 5 die Darstellung der Überschneidungsbereiche, in denen Schließkörper und Klappenring kollidieren würden, wenn nicht entsprechende Aussparungen vorgesehen wären,

Fig. 6 einen Schnitt durch eine Lagerstelle und

Fig. 7 das Montieren des Schließkörpers im Klappenring.

Das nachfolgend beschriebene Ausführungsbeispiel ist eine zweiflügelige Herzklappenprothese. Diese weist einen kreisringförmigen Klappenring 10 auf, in dem zwei Schließkörper 11 in Form gebogener Platten jeweils um eine Schwenkachse 12 herum schwenkbar sind. Der Klappenring 10 weist an seiner Außenseite eine umlaufende Nut auf, in der ein Nahtring 13 sitzt, um die Herzklappenprothese am Körpergewebe annähen zu können. Die Durchflußrichtung während der Systole ist durch den Pfeil 14 bezeichnet.

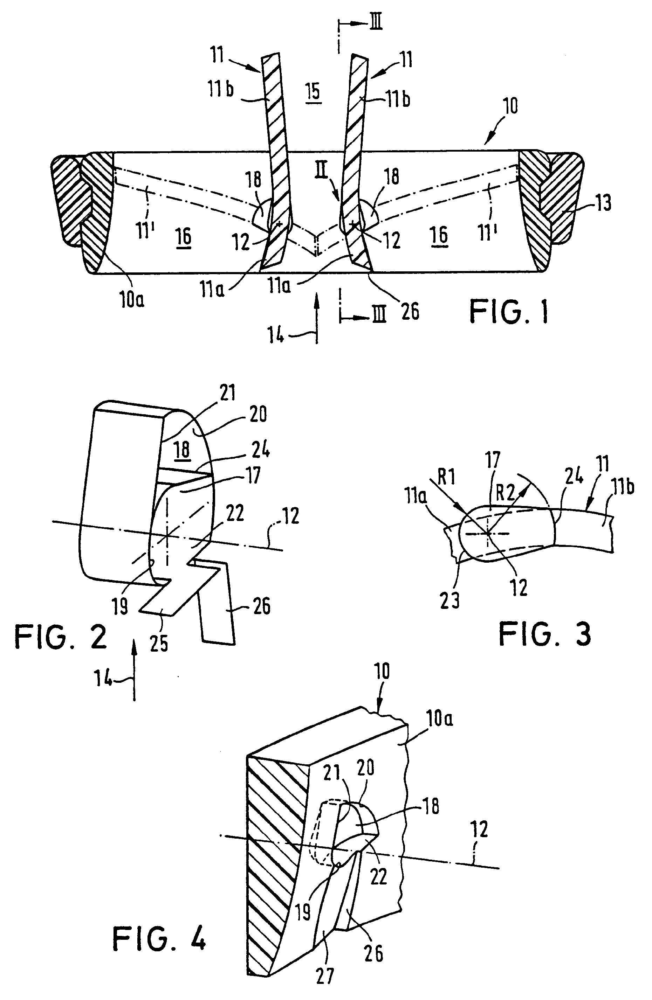

Die Innenfläche 10a des Klappenringes 10 verengt sich in Durchflußrichtung vom Einlaß bis zum Auslaß stetig. Jeder der Schließkörper 11 hat eine annähernd halbkreisförmige Gestalt und er bildet - bezogen auf die Schwenkachse 12 - einen zweiarmigen Hebel. Die kürzeren Teile 11a der Schließkörper stoßen in der Schließstellung mit ihren Kanten in der Mittelebene des Ringes 10 zusammen, während sich die längeren Teile 11b mit ihren umlaufenden Kanten abdichtend gegen die Innenseite 10a legen. Die Schließstellung der Schließkörper 11 ist in Fig. 1 gestrichelt angegeben. In der Öffnungsstellung begrenzen die Schließkörper 11 einen düsenförmigen Kanal 15 und jeder Schließkörper begrenzt zusammen mit der Innenfläche 10a einen annähernd halbkreisförmigen Kanal 16. Durch den Blutdruck in der Diastole werden die Schließkörper 11 in die Schließposition gebracht, während sie durch den systolischen Druck in Richtung des Pfeiles 14 geöffnet werden.

Jeder Schließkörper 11 weist entlang seiner Schwenkachse 12 zwei nach entgegengesetzten Richtungen abstehende Lagerzapfen 17 auf, von denen jeder in eine Ausnehmung 18 des Klappenringes 10 hineinragt.

Wie aus den Fign. 2 und 3 hervorgeht, hat die Ausnehmung 18 die Form eines Kreissektors, wobei der den Sektorwinkel bildende Innenbereich 19 kreisbogenförmig abgerundet ist. Der Umfangs- oder Außenbereich 20 des Kreissektors bildet eine konkave Fläche, die der konkaven Fläche des Bereichs 19 entgegengerichtet ist. Die radiale Begrenzungswand 21 der Ausnehmung 18 verläuft im wesentlichen parallel zur Strömungsrichtung (Pfeil 14), während die andere Begrenzungswand 22 etwa in der Richtung verläuft, die der Schließkörper in der Schließstellung 11' einnimmt. Der Winkel zwischen den Begrenzungswänden 21 und 22 beträgt etwa 70°. Er entspricht annähernd dem Schwenkwinkel des Schließkörpers 11.

In der Ausnehmung 18 sitzt der Lagerzapfen 17, der eine halbzylindrische erste Stützfläche 23 und eine kreisbogenförmige zweite Stützfläche 24 aufweist. Die erste Stützfläche 23 ist um die Schwenkachse 12 mit dem Radius R1 gebogen und die zweite Stützfläche 24 ist um die Schwenkachse 12 mit dem Radius R2 gebogen. Die Schwenkachse 12 befindet sich zwischen den Stützflächen 23 und 24. Der Radius R2 beträgt etwa das Doppelte des Radius R1. Der Lagerzapfen 17 hat längliche Form, wobei die Stützfläche 23 zum Teil 11a und die Stützfläche 24 zum Teil 11b des Schließkörpers weist.

Der in der Ausnehmung 18 sitzende Lagerzapfen 17 stützt sich mit der Stützfläche 23 an dem Innenbereich 19 ab, während die Stützfläche 24 an dem Außenbereich 20 anliegt. Der Lagerzapfen 17 ist um die Schwenkachse 12 herum schwenkbar, wobei er in der Öffnungsstellung an der Radialwand 21 und in der Schließstellung gemäß Fig. 2 an der Radialwand 22 der Ausnehmung 18 anliegt. Die Stützfläche 23 wälzt sich bei der Schwenkbewegung an dem Innenbereich 19 ab, während die Stützfläche 24 sich entlang des Außenbereichs 20 bewegt. Da der Lagerzapfen 17 die Ausnehmung 18 nicht ausfüllt, führt er bei jeder Schwenkbewegung einen Verdrängungsvorgang aus, durch den das in der Ausnehmung 18 befindliche Blut verdrängt und die Aussparung durchspült wird.

In Fig. 2 ist schematisch in Verbindung mit dem Lagerzapfen 17 eine Anschlagfläche 25 dargestellt, die mit einer Anschlagfläche 26 des Klappenringes 10 zusammenwirkt, um die Öffnungsbewegung des Schließkörpers zu begrenzen. Die Realisierung der Anschlagfläche 26 ist in Fig. 4 dargestellt. Diese Anschlagfläche bildet das Ende einer Aussparung 27, die sich in der Innenfläche 10a von der Ausnehmung aus stromauf erstreckt und die einerseits dazu dient, Kollisionen zwischen der Schließkörperkante und dem Klappenring bei der Öffnungsbewegung zu vermeiden, und andererseits eine einfache Realisierung der Anschlagfläche 26 ermöglicht. Ein weiterer Zweck der Aussparung 27 besteht darin, die Montage des Schließkörpers im Klappenring zu erleichtern.

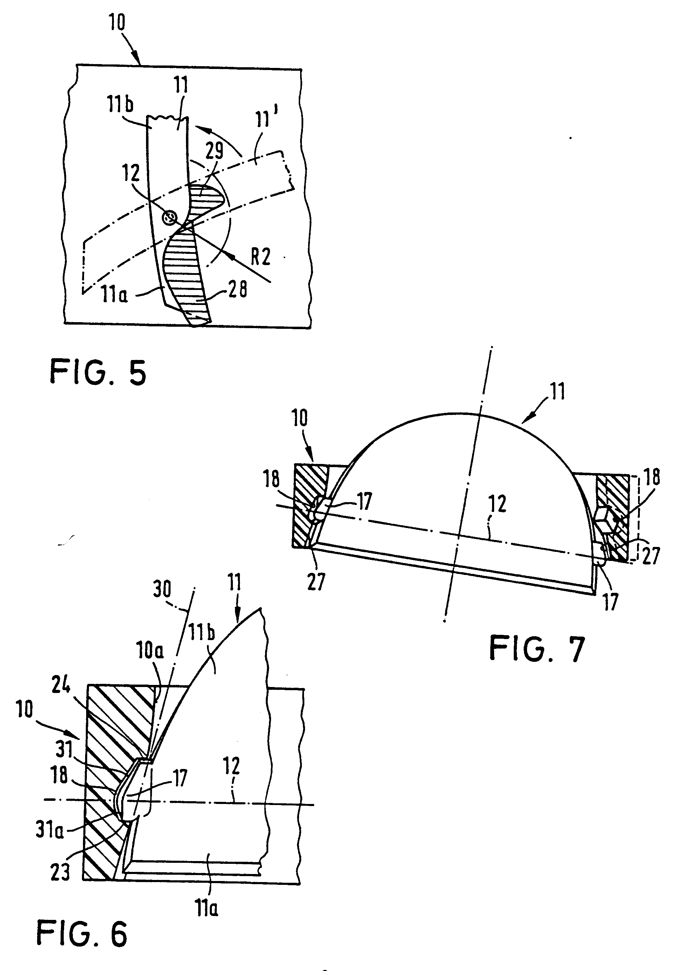

In Fig. 5 ist die Position des Schließkörpers 11 innerhalb des Klappenringes 10 in der Öffnungsstellung in durchgezogenen Linien und in der Schließstellung gestrichelt dargestellt. Die schraffierten Bereiche 28 und 29 geben die Überschneidungsbereiche an, in denen sich der Rand des Schließkörpers mit der Innenfläche 10a des Klappenringes unter Einhaltung eines bestimmten, aus Dichtigkeitsgründen nicht zu überschreitenden radialen Spiels überschneidet, wenn keine zusätzlichen Maßnahmen ergriffen werden. Die Maßnahme zur Vermeidung der Überschneidungsfläche 28 besteht darin, die Aussparung 27 am Klappenring 10 vorzusehen. Die Maßnahme zur Vermeidung der Überschneidungsfläche 29 in der Schließstellung des Schließkörpers besteht darin, daß der Radius R2 der Stützfläche 24 bzw. der Radius des Außenbereichs 20, bezogen auf die Schwenkachse 12, mindestens die Größe des Überschneidungsbereichs 29 hat. Mit anderen Worten: Wenn der Lagerzapfen 17 nicht vorhanden wäre, würde die Umfangskante des Schließkörpers 11 in der Schließstellung geringfügig in die Ausnehmung 18 eintauchen.

Wie aus Fig. 6 hervorgeht, fallen in der Öffnungsstellung die Tangenten 30 an die Innenfläche 10a des Klappenringes und an die Umfangskante des Schließkörpers 11 in der Drehachse 12 zusammen. Dadurch werden die Größen der in Fig. 5 dargestellten Schnittflächen 28 und 29 minimiert.

Da die Innenseite des Klappenringes entgegen der Strömungsrichtung divergiert, sind der Innenbereich 19 und der Außenbereich 20 der sektorförmigen Ausnehmung 18 in Richtung der Schwenkachse 12 gegeneinander versetzt und demgemäß liegt die Stützfläche 24 näher an der Ringachse als die Stützfläche 23. In dem an die Stützfläche 24 angrenzenden Bereich verläuft die Zapfenendfläche 31 konisch schräg nach außen. Der im Bereich der Schwenkachse 12 gelegene Abschnitt 31a der Zapfenendfläche ist sphärisch ausgebildet und geht tangential in den konischen Abschnitt über. Die gesamte Zapfenendfläche 31 entspricht dabei dem Ausschnitt einer Rotationsfläche um die Drehachse 12, weil die Wischbewegung des Lagerzapfens 17 auf eine Drehung beschränkt ist.

Wie aus Fig. 6 ferner hervorgeht, ist der Verlauf der Zapfenendfläche 31 auf den Verlauf des Grundes der Ausnehmung 18 so abgestimmt, daß beide unter Einhaltung eines Spiels parallel zueinander verlaufen. Daher wird bei einer Schwenkbewegung des Schließkörpers 11 die Ausnehmung 18 von dem Lagerzapfen 17 vollständig überstrichen und ausgewaschen.

Fig. 7 zeigt das Einsetzen des Schließkörpers 11 in den Klappenring 10. Hierbei wird zunächst ein Lagerzapfen 17 in die entsprechende Ausnehmung 18 eingesetzt und der andere Lagerzapfen 17 wird von dem stromauf gerichteten Ende her, mit dem konischen Abschnitt der Zapfenendfläche 31 in der Aussparung 27 gleitend, in die entsprechende Ausnehmung 18 eingeschoben. Hierbei erfolgt kurzzeitig eine geringfügige Aufweitung des Klappenringes, so wie dies in Fig. 7 gestrichelt angedeutet ist.

**Patentansprüche**

1. Herzklappenprothese mit einem Klappenring (10), dessen Innenfläche (10a) sich in Strömungsrichtung zunehmend verengt, und mindestens einem Schließkörper (11), der Lagerzapfen (17) aufweist, welche in Ausnehmungen (18) eingreifen, die im Verengungsbereich des Klappenringes (10) angeordnet sind,
**dadurch gekennzeichnet,**
daß die Ausnehmungen (18) die Form eines Kreissektors mit Kreisförung abgerundetem Innenbereich (19) haben und daß die Lagerzapfen (17) länglich sind und mit einer dem abgerundeten Innenbereich (19) angepaßten und sich bei ber Schwenkbewegung des Schließkörpers darin abwäbzenden ersten Stützfläche (23) an dem Innenbereich (19) und mit einer zweiten Kreisbogenförmigen Stützfläche (24) am Außenbereich (20) des Kreissektors stets angreifen, wobei die Drehachse (12) mit der Krümmungsachse der ersten Stützfläche (23) zusammenfällt.

2. Herzklappenprothese nach Anspruch 1, dadurch gekennzeichnet, daß die Tangente (30) an die Innenfläche des Klappenringes in der Drehachse (12) und die Tangente an die Umfangskante des Schließkörpers in der Drehachse (12) annähernd zusammenfallen.

3. Herzklappenprothese nach Anspruch 1 oder 2, dadurch gekennzeichnet, daß der Radius (R2) der zweiten Stützfläche (24) - bezogen auf die Drehachse (12) - wesentlich größer ist als derjenige der ersten Stützfläche (23), während die Umfangserstreckung der zweiten Stützfläche (24) kleiner ist als diejenige der ersten Stützfläche (23).

4. Herzklappenprothese nach einem der Ansprüche 1 bis 3, dadurch gekennzeichnet, daß die stromauf gerichtete erste Stützfläche (23) gegenüber der stromab gerichteten zweiten Stützfläche (24) nach außen versetzt ist.

5. Herzklappenprothese nach einem der Ansprüche 1 bis 4, dadurch gekennzeichnet. daß der parallel zur Drehachse (12) gerichtete Abstand zwischen den Zapfenendflächen (31) der Lagerzapfen (17) zur zweiten Stützfläche (24) hin kontinuierlich abnimmt.

6. Herzklappenprothese nach einem der Ansprüche 1 bis 5, dadurch gekennzeichnet, daß die Endfläche (31) des Lagerzapfens (17) den Grund der Ausnehmung (18) an allen Stellen mit geringem Abstand überstreicht.

7. Herzklappenprothese nach einem der Ansprüche 1 bis 6, dadurch gekennzeichnet, daß der Grund der Ausnehmung (18) als Ausschnitt einer Rotationsfläche um die Drehachse (12) ausgebildet ist.

8. Herzklappenprothese nach einem der Ansprüche 1 bis 7, dadurch gekennzeichnet, daß der Grund der Ausnehmung (18) im Bereich der Drehachse (12) sphärisch und stromab der Drehachse (12) konisch ausgebildet ist.

9. Herzklappenprothese nach einem der Ansprüche 1 bis 8, dadurch gekennzeichnet, daß stromauf der Ausnehmung (18) im Klappenring (10) eine die Schließkörperkante in der Öffnungsstellung aufnehmende Aussparung (27) mit einer Anschlagfläche (26) zur Begrenzung der Öffnungsstellung vorgesehen ist.

## Claims

1. A heart valve prosthesis comprising a valve ring (10) the inner surface (10a) of which converges progressively in flow direction, and at least one closing member (11) provided with journal pins (17) engaging in recesses (18) arranged in the constricted portion of said valve ring (10),
characterised in that
each of said recesses (18) is provided in the shape of a sector with a circularly rounded inner portion (19) and that said journal pins (17) are oblong in cross-section and permanently engage the inner portion (19) of the sector with a first supporting surface (23) adapted to said rounded inner portion (19) and rolling therein upon a pivotal movement of said closing member, and the outer portion (20) of said sector with a second sector-shaped supporting surface (24), the swivel axis (12) coinciding with the axis of the curvature of the first supporting surface (23).

2. The heart valve prosthesis according to claim 1, characterised in that a tangent line (30) to the inner surface of said valve ring at said swivel axis (12) and a tangent line at the peripheral edge of said closing member at said swivel axis (12) approximately coincide.

3. The heart valve prosthesis according to claim 1 or 2, characterised in that the radius (R2) of said second supporting surface (24) - relative to said swivel axis (12) - is substantially larger than that of said first supporting surface (23), whereas the peripheral extension of said second surface (24) is smaller than that of the first supporting surface (23).

4. The heart valve prosthesis according to one of claims 1 to 3, characterised in that said upstream-directed first supporting surface (23) is outwardly offset with respect to said downstream-directed second supporting surface (24).

5. The heart valve prosthesis according to one of claims 1 to 4, characterised in that the distance between the end surfaces (31) of the journal pins (17) and said second supporting surface (24) - in a direction parallel to said swivel axis (12) - decreases continuously.

6. The heart valve prosthesis according to one of claims 1 to 5, characterised in that the end surface (31) of said journal pin (17) sweeps the entire bottom of said recess (18) in a short distance thereto.

7. The heart valve prosthesis according to one of claims 1 to 6, characterised in that the bottom of said recess (18) is provided as a section of a surface of rotation around said swivel axis (12).

8. The heart valve prosthesis according to one of claims 1 to 7, characterised in that the bottom of said recess (18) is shaped spherically in the vicinity of said swivel axis (12) and conically downstream of said swivel axis (12).

9. The heart valve prosthesis according to one of claims 1 to 8, characterised in that upstream of said recess (18) a second recess (27) is provided in said valve ring (10) for receiving the edge of said closing member in the closed position, said second recess (27) having an abutment (26) for limiting the open position.

## Revendications

1. Prothèse de valve cardiaque comportant un anneau de valve (10) dont la surface intérieure (10a) se rétrécit de manière croissante dans la direction de l'écoulement, et au moins un corps de fermeture (11) pourvu de tourillons de palier (17) qui s'engagent dans des évidements (18) disposés dans la zone de rétrécissement de l'anneau de valve (10),

caractérisée en ce que

les évidements (18) présentent une forme d'un secteur circulaire comportant une zone intérieure (19) arrondie de manière circulaire, et en ce que les tourillons de palier (17) sont allongés et s'appliquent continuellement contre la zone intérieure (19) par l'intermédiaire d'une première surface d'appui (23) adaptée à la zone intérieure (19) arrondie et roulant sur celle-ci lors du mouvement de pivotement du corps de fermeture, et par l'intermédiaire d'une seconde surface d'appui (24) en forme d'arc de cercle, contre la zone extérieure (20) du secteur circulaire, l'axe de rotation (12) se confondant avec l'axe de courbure de la première surface d'appui (23).

2. Prothèse de valve cardiaque selon la revendication 1, caractérisée en ce que la tangente (30) à la surface intérieure de l'anneau de valve au niveau de l'axe de rotation (12), et la tangente au bord périphérique du corps de fermeture au niveau de l'axe de rotation (12), sont approximativement confondues.

3. Prothèse de valve cardiaque selon la revendication 1 ou 2, caractérisée en ce que le rayon (R2) de la seconde surface d'appui (24), - rapporté à l'axe de rotation (12), est sensiblement plus grand que celui de la première surface d'appui (23), tandis que l'étendue périphérique de la seconde surface d'appui (24) est plus faible que celle de la première surface d'appui (23).

4. Prothèse de vaîve cardiaque selon l'une des revendications 1 à 3, caractérisée en ce que la première surface d'appui (23) dirigée vers l'amont est décalée vers l'extérieur par rapport à la seconde surface d'appui,(24) dirigée vers l'aval.

5. Prothèse de valve cardiaque selon l'une des revendications 1 à 4, caractérisée en ce que la distance orientée parallèlement à l'axe de rotation (12), entre les surfaces d'extrémité de tourillon (31) des tourillons de palier (17), diminue continuellement en direction de la seconde surface d'appui (24).

6. Prothèse de valve cardiaque selon l'une des revendications 1 à 5, caractérisée en ce que la surface d'extrémité (31) du tourillon de palier (17) balaye le fond de l'évidement (18) à faible distance de celui-ci en tous les points.

7. Prothèse de valve cardiaque selon l'une des revendications 1 à 6, caractérisée en ce que le fond de l'évidement (18) est réalisé sous la forme d'un tronçon de surface de révolution autour de l'axe de rotation (12).

8. Prothèse de valve cardiaque selon l'une des revendications 1 à 7, caractérisée en ce que le fond de l'évidement (18) est de configuration sphérique dans la région de l'axe de rotation (12), et conique en aval de l'axe de rotation (12).

9. Prothèse de valve cardiaque selon l'une des revendications 1 à 8, caractérisée en ce qu'en amont de l'évidement (18), dans l'anneau de valve (10), est prévue une encoche (27) destinée à loger le bord du corps de fermeture dans la position d'ouverture, et comportant une surface de butée (26) pour limiter la position d'ouverture.

FIG. 1

FIG. 2

FIG. 3

FIG. 4

FIG. 5

FIG. 7

FIG. 6

8